(19) Europäisches Patentamt
European Patent Office
Office européen des brevets

(11) Numéro de publication: **0 299 892**
**A1**

(12) **DEMANDE DE BREVET EUROPEEN**

(21) Numéro de dépôt: **88420244.1**

(51) Int. Cl.[4]: **C 07 C 37/62**

(22) Date de dépôt: **13.07.88**

(30) Priorité: **17.07.87 FR 8710419**

(43) Date de publication de la demande:
**18.01.89 Bulletin 89/03**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevoie Cédex (FR)**

(72) Inventeur: **Desmurs, Jean-Roger**
**La Jonquière Route de Ternay**
**Communay F-69360 St.-Symhorien D'Ozon (FR)**

**Besson, Bernard**
**15, rue de Moucherotte**
**F-38800 Pont de Claix (FR)**

**Jouve, Isabelle**
**30, rue Léon Fabre**
**F-69100 Villeurbanne (FR)**

(74) Mandataire: **Vignally, Noel et al**
**RHONE-POULENC INTERSERVICES Service Brevets**
**Chimie Centre de Recherches de Saint-Fons B.P. 62**
**F-69192 Saint-Fons Cedex (FR)**

(54) **Procédé de préparation de trichloro-2,4,6 phénol.**

(57) La présente invention concerne la chloration de divers chlorophénols par le chlore gazeux, pour préparer de manière sélective le trichloro-2,4,6 phénol.

Plus précisément elle consiste en un procédé de préparation de trichloro-2,4,6 phénol, par chloration par le chlore gazeux, d'orthochlorophénol et/ou de parachlorophénol et/ou de dichloro-2,4 phénol et/ou de dichloro-2,6 phénol, caractérisé en ce que l'on opère en présence :
- d'une quantité efficace d'un cation organique ou d'une amine primaire, secondaire ou tertiaire ;
- et d'une quantité efficace d'un sulfure organique.

Le trichloro-2,4,6 phénol est un intermédiaire organique servant notamment pour la synthèse de produits phytosanitaires et de colorants.

EP 0 299 892 A1

**Description**

## PROCEDE DE PREPARATION DE TRICHLORO-2,4,6 PHENOL

La présente invention concerne la chloration de divers chlorophénols par le chlore gazeux, pour préparer de manière sélective le trichloro-2,4,6 phénol.

Le trichloro-2,4,6 phénol est un intermédiaire organique servant notamment pour la synthèse de produits phytosanitaires et de colorants.

L'obtention de chlorophénols par chloration du phénol ou de chlorophénols moins chlorés est connue depuis très longtemps.

Mais on obtient aux différents stades de la chloration des isomères ; cela conduit à de nombreux composés qu'il est ensuite nécessaire de séparer par des opérations coûteuses et délicates.

En outre, au fur et à mesure de l'avancement du stade de chloration, la fixation de nouveaux atomes de chlore sur le cycle benzénique devient plus difficile. Ainsi au stade trichlorophénol, il se forme déjà des quantités non négligeables de chlorocyclohexadiénones, composés instables conduisant à de nombreux sous-produits indésirables.

La présente invention se propose de résoudre le problème de la chloration sélective en trichloro-2,4,6 phénol de l'orthochlorophénol et/ou du parachlorophénol et/ou du dichloro-2,4 phénol et/ou du dichloro-2,6 phénol.

Plus précisément elle consiste en un procédé de préparation de trichloro-2,4,6 phénol, par chloration par le chlore gazeux, d'orthochlorophénol et/ou de parachlorophénol et/ou de dichloro-2,4 phénol et/ou de dichloro-2,6 phénol, caractérisé en ce que l'on opère en présence :
- d'une quantité efficace d'un cation organique ou d'une amine primaire, secondaire ou tertiaire ;
- et d'une quantité efficace d'un sulfure organique.

On peut mettre en oeuvre un cation organique ou un mélange de plusieurs cations organiques.

Par cation organique on entend dans le présent texte les ions onium dérivant notamment de l'azote, du phosphore, de l'arsenic, du soufre, du sélénium, de l'oxygène, du carbone ou de l'iode et coordiné à des restes hydrocarbonés. Les ions onium dérivant de l'azote, du phosphore ou de l'arsenic seront quadricoordinés, les ions onium dérivant du soufre, du sélénium, de l'oxygène, du carbone ou de S=O seront tricoordinés tandis que les ions onium dérivant de l'iode seront dicoordinés.

Les restes hydrocarbonés coordinés à ces différents éléments sont des radicaux alkyles, alcényles, aryles, cycloalkyles, aralkyles éventuellement substitués, 2 restes hydrocarbonés coordinés pouvant former ensemble un radical unique divalent.

La nature des anions liés à ces cations organiques n'a pas d'importance critique. Toutes les bases "dures" ou "intermédiaires" conviennent comme anion.

Par base "dure" ou "intermédiaire", on entend tout anion répondant à la définition classique donnée par R. PEARSON dans Journal of Chem. Ed. 45, pages 581 - 587 (1968), les termes "dure" et "intermédiaire" ayant respectivement la signification des termes de "hard" et "borderline" utilisés dans cette référence.

Parmi les ions onium pouvant être utilisés dans le présent procédé de chloration, conviennent particulièrement ceux répondant à l'une des formules générales suivantes :

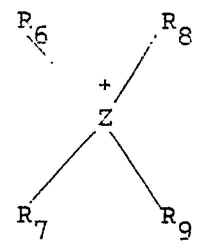

(I)

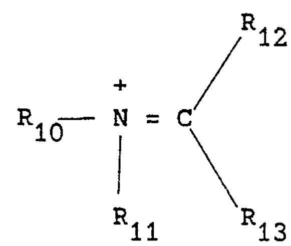

(II)

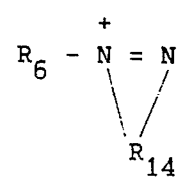

(II bis)

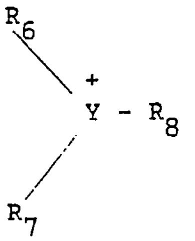

(III)

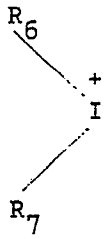

(IV)

dans lesquelles :

- Z représente N, P ou As ;
- Y représente S, O, Se, S=O ou C ;
- $R_6$, $R_7$, $R_8$ et $R_9$, identiques ou différents représentent :

. un radical alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;

. un radical alcényle, linéaire ou ramifié, ayant 2 à 12 atomes de carbone ;

. un radical aryle ayant 6 à 10 atomes de carbone, éventuellement substitué par un ou plusieurs groupements ou atomes alkyles ayant 1 à 4 atomes de carbone, alkoxy, alkoxycarbonyle, le radical alkoxy ayant 1 à 4 atomes de carbone, ou halogène,

. deux desdits radicaux $R_6$ à $R_9$ pouvant former ensemble un radical alkylène, alcénylène ou alcadiènylène, linéaire ou ramifié ayant de 3 à 6 atomes de carbone ;

- $R_{10}$, $R_{11}$, $R_{12}$, $R_{13}$ sont identiques ou différents et représentent :

. un radical alkyle linéaire ou ramifié contenant de 1 à 4 atomes de carbone ;

. les radicaux $R_{12}$ et $R_{13}$ pouvant former ensemble un radical alkylène contenant de 3 à 6 atomes de carbone ;

. les radicaux $R_{11}$ et $R_{12}$ ou $R_{11}$ et $R_{13}$ pouvant former ensemble un radical alkylène, alcénylène ou alcadiènylène, contenant 4 atomes de carbone et constituant avec l'atome d'azote un hétérocycle azoté ;

- $R_{14}$ représente un radical divalent formant avec les 2 atomes d'azote un cycle ayant 4 à 6 atomes pouvant comporter un ou plusieurs atomes d'azote, de soufre et/ou d'oxygène ledit cycle pouvant être substitué par un ou plusieurs radicaux tels que $R_6$.

Parmi les bases "dures" ou "intermédiaires" pouvant constituer l'anion desdits sels d'onium, on peut citer les ions : $F^-$, $ClO_4^-$ , $PF_6^-$ , $BF_4^-$ , $SnCl_6^-$ , $SbCl_6^-$ , $B(Ph)_4^-$ , $PO_4^{3-}$ , $HPO_4^{2-}$ , $H_2PO_4^-$ , $CH_3SO_3^-$ , $Ph\text{-}SO_3^-$ , $HSO_4^-$ , $NO_3^-$ , $SO_4^{2-}$ , $Cl^-$, $Br^-$, $I^-$, $OH^-$, Ph représentant un radical phényl, ainsi que tous les autres anions répondant à la définition de base "dure" ou "intermédiaire" de PEARSON.

Pour des raisons de commodité de mise en oeuvre, lesdits anions pourront être choisis parmi $PO_4^{3-}$ , $HPO_4^{2-}$ , $H_2PO_4^-$ , $CH_3SO_3^-$ , $Ph\text{-}SO_3^-$ ,$NO_3^-$ , $SO_4^{2-}$ , $PF_6^-$ , $Cl^-$, $Br^-$, $I^-$, Ph ayant la signification précédente. On recourra avantageusement aux anions $Cl^-$ et $Br^-$ et plus particulièrement à l'anion $Cl^-$.

A titre d'exemples de cations organiques répondant à la formule (I) on peut citer les cations :

- tétraméthylammonium,
- triéthylméthylammonium,
- tributylméthylammonium,
- triméthylpropylammonium,
- tétraéthylammonium,
- tétrabutylammonium,
- dodécyltriméthylammonium,
- méthyltrioctylammonium,
- heptyltributylammonium,
- tétrapropylammonium,
- tétrapentylammonium,
- tétrahexylammonium,
- tétraheptylammonium,
- tétraoctylammonium,
- tétradécylammonium,
- butyltripropylammonium,
- méthyltributylammonium,
- pentyltributylammonium,
- méthyldiétylpropylammonium,
- éthyldiméthylpropylammonium,
- tétradodécylammonium,
- tétraoctadécylammonium,
- hexadécyltriméthylammonium,
- benzyltriméthylammonium,
- benzyldiméthylpropylammonium,
- benzyldiméthyloctylammonium,
- benzyltributylammonium,
- benzyltriéthylammonium,
- phényltriméthylammonium,
- benzyldiméthyltétradécylammonium,
- benzyldiméthylhexadécylammonium,
- diméthyldiphénylammonium,
- méthyltriphénylammonium,
- butène-2-yltriéthylammonium,
- N,N-diméthyl-tétraméthylèneammonium,
- N,N-diéthyl-tétraméthylènammonium,
- tétraméthylphosphonium,
- tétrabutylphosphonium,
- éthyltriméthylphosphonium,
- triméthylpentylphosphonium,
- octyltriméthylphosphonium,
- dodécyltriméthylphosphonium,
- triméthylphénylphosphonium,
- diéthyldiméthylphosponium,
- dicyclohexeyldiméthylphosphonium,
- diméthyldiphénylphosphonium,
- cyclohexyltriméthylphosphonium,
- triéthylméthylphosphonium,
- méthyltri(isopropyl)phosphonium,
- méthyltri(n-propyl)phosphonium,
- méthyltri(n-butyl)phosphonium,
- méthyltri(méthyl-2 propyl)phosphonium,
- méthyltricyclohexylphosphonium,
- méthyltriphénylphosphonium,
- méthyltribenzylphosphonium,

- méthyltri(méthyl-4 phényl) phosphonium,
- méthyltrixylylphosphonium,
- diéthylméthylphénylphosphonium,
- dibenzylméthylphénylphosphonium,
- éthyltriphénylphosphonium,
- tétraéthylphosphonium,
- éthyltri(n-propyl)phosphonium,
- triéthylpentylphosphonium,
- hexadécyltributylphosphonium,
- éthyltriphénylphosphonium,
- n-butyltri(n-propyl)phosphonium,
- butyltriphénylphosphonium,
- benzyltriphénylphosphonium,
- (β-phényléthyl)diméthylphénylphosphonium,
- tétraphénylphosphonium,
- triphényl(méthyl-4 phényl)phosphonium,
- tétrakis(hydroxyméthyl)phosphonium,
- tétraphénylarsonium.

Parmi les cations répondant aux formules (II) et (II bis) on peut citer les cations :
- N-méthylpyridinium,
- N-éthylpyridinium,
- N-hexadécylpyridinium,
- N-méthylpicolinium,
- triphényl-1,2,4 triazolium.

A titre d'exemples de cations organiques répondant à la formule (III), on peut citer les cations :
- triméthylsulfonium,
- triéthylsulfonium,
- triphénylsulfonium,
- triméthylsulfoxonium,
- triphénylcarbénium,
- triéthyloxonium.

A titre d'exemples de cations organiques répondant à la formule (IV), on peut citer les cations :
- diphényliodonium,
- diméthoxy-4,4′ diphényliodonium (ou les composés décrits dans JACS 1958, 81, 342),
- diphényliodonium 2-carboxylate

Parmi les cations organiques qui peuvent être utilisés dans le cadre du présent procédé, on préfèrera le plus souvent les ions ammonium quaternaire, les ions phosphonium quaternaire, les ions sulfonium et les ions iodonium.

Le sel d'onium peut être introduit à l'état solide ou sous forme d'une solution dans l'un de ses solvants, le plus souvent l'eau.

Le procédé selon l'invention peut être conduit en l'absence de solvant : les réactifs sont alors à l'état fondu. Cette variante de l'invention conduit généralement aux meilleures résultats.

Lorsque l'on utilise un cation organique, on peut également opérer dans un milieu liquide constitué notamment par un éther aliphatique, un hydrocarbure aliphatique, un hydrocarbure aliphatique chloré, un chlorobenzène ou un bromobenzène, un acide carboxylique.

A titre d'éther aliphatique, on peut citer notamment l'oxyde de dipropyle, l'oxyde de diisopropyle, l'oxyde de méthyle et de tertiobutyle.

A titre d'hydrocarbure aliphatique, on peut citer notamment l'hexane, l'heptane, l'octane, le nonane et le décane.

A titre d'hydrocarbure chloré, on peut citer les hydrocarbures perchlorés tels que notamment le tétrachlorure de carbone, le tétrachloroéthylène, l'hexachloroéthane, l'hexachloropropène et l'hexachlorométhylène; les hydrocarbures partiellement chlorés tels que le chlorure de méthylène, le dichloroéthane, le tétrachloroéthane, le trichloroéthylène, le chloro-1 butane, le dichloro-1,2 butane.

A titre de chlorobenzène, on peut citer en particulier le monochlorobenzène, le dichloro-1,2 benzène, le dichloro-1,3 benzène, le dichloro-1,4 benzène ou des mélanges de différents chlorobenzènes ; à titre de bromobenzène, on peut citer notamment le monobromobenzène ou des mélanges de monobromobenzène avec un ou plusieurs dibromobenzènes.

A titre d'acide carboxylique, on peut citer plus particulièrement l'acide acétique et l'acide propionique.

Lorsque le procédé de l'invention est mis en oeuvre en milieu solvant, la concentration du chlorophénol dans le solvant n'est pas critique. Elle dépendra notamment de la solubilité du chlorophénol dans le solvant utilisé.

La quantité de cation organique utilisée dans le procédé peut varier dans de très larges limites.

Habituellement elle représente en poids de sel d'onium par rapport au poids du chlorophénol à chlorer de 0,005 % à 25 %.

Lorsque l'on opère à l'état fondu, on mettra en oeuvre préférentiellement de 0,015 % à 5 % en poids de sel d'onium par rapport au chlorophénol à chlorer, afin d'avoir une efficacité suffisante, tout en n'ayant par une quantité excessive de sel d'onium.

Lorsque l'on opère en milieu solvant, on mettra en oeuvre préférentiellement de 5 à 25 % en poids de sel d'onium par rapport au chlorophénol. Dans ce cas il est préférable d'exprimer la quantité de sel d'onium par rapport au mélange réactionnel. On utilisera par exemple de 0,01 % à 2 % en poids de sel d'onium par rapport au mélange réactionnel.

Par amine, on entend dans le présent texte tout composé, liquide ou solide dans les conditions opératoires du procédé, comportant une ou plusieurs fonctions amine.

Un tel composé peut également comporter une ou plusieurs autres fonctions chimiques, telles que par exemple la fonction hydroxyle, la fonction acide carboxylique, la fonction ester d'acide carboxylique, la fonction amide ou la fonction imine.

Il est bien évident que les amines utilisées peuvent être introduites également sous la forme de leurs sels et plus particulièrement de leurs chlorhydrates respectifs.

Dans le présent texte, le terme "amine" englobe également l'ammoniac ainsi que les sels, notamment les chlorhydrates d'amines.

Les amines utilisées comme catalyseur dans le présent procédé sont plus particulièrement les amines de formule générale (V) :

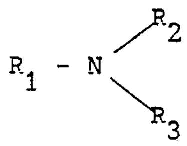

(V)

dans laquelle :

- $R_1$, $R_2$ et $R_3$, identiques ou différents, représentent :

. un radical alklye linéaire ayant de 1 à 12 atomes de carbone, un radical alkyle secondaire ayant 3 à 12 atomes de carbone ou un radical alkyle tertiaire ayant 4 à 12 atomes de carbone, ces radicaux alukyles pouvant comporter une ou deux fonctions éther -O- ou fonctions hydroxyle, amine, acide carboxylique, ester d'acide carboxylique, amide ou imine ;

. un radical phényl, un radical cyclohexyle, un radical cycloheptyle ou un radical cyclopentyle ;

. un radical phénylalkyle, cyclohexylalkyle, cycloheptylalkyle ou cyclo pentylalkyle dont la partie alkyle comporte 1 à 4 atomes de carbone ;

. un atome d'hydrogène.

- $R_1$ peut représenter un groupement $NH_2$ ;

- $R_2$ et $R_3$ forment ensemble et avec l'atome d'azote un hétérocycle, saturé ou comportant une ou plusieurs doubles liaisons, éventuellement substitué par un ou plusieurs groupements alkyles ayant 1 à 4 atomes de carbone ;

- $R_2$ et $R_3$ ou $R_1$, $R_2$ et $R_3$ forment ensemble, avec l'atome d'azote et avec un ou plusieurs atomes d'azote et/ou d'oxygène et/ou de soufre, un hétérocycle, saturé ou insaturé, éventuellement substitué par un ou plusieurs groupements alkyles ayant 1 à 4 atomes de carbone ;

- $R_1$, $R_2$ et $R_3$ forment ensemble et avec l'atome d'azote un hétérocycle insaturé éventuellement substitué par un ou deux groupements méthyle ou éthyle ;

- $R_2$ et $R_3$ ou $R_1$, $R_2$ et $R_3$ forment ensemble, avec l'atome d'azote et éventuellement avec un ou plusieurs atomes d'azote et/ou d'oxygène et/ou de soufre, un composé polycyclique, saturé ou insaturé, éventuellement substitué par un ou plusieurs groupements alkyles ayant 1 à 4 atomes de carbone.

A titre d'exemples illustratifs, on peut citer comme amines de formule (V) :

- l'ammoniac ;

- des amines primaires telles que la n-propylamine, l'isopropylamine, l'isobutylamine, la n-butylamine, la tertiobutylamine, la n-pentyl amine, la méthyl-2 butylamine, la méthyl-3 butylamine, la n-hexylamine, l'éthyl-2 hexylamine, l'aniline, la laurylamine, la cyclohexylamine, la cyclopentylamine, la benzylamine, la guanidine, l'acétamidine, l'ester éthylique de la glycine, l'éthanolamine, l'éthylènediamine, l'hexaméthylènediamine, la N-aminoéthylpyrrolidine, la pyrazoline, la lysine, la N-aminomorpholine, la N-aminopipéridine ;

- des amines secondaires telles que la dibutylamine, la dipropylamine, la méthylpropylamine, la méthylbutylamine, la méthylisobutylamine, la méthyltertiobutylamine, la méthylbenzylamine, la ditertiobutylamine, la méthyl-1 cyclopentylamine, la méthyl-1 cyclohexylamine, la dicyclohexylamine, la morpholine, l'amidazole, la pyrrolidine, l'imidazolidine, la pipérazine, l'indole ;

- des amines tertiaires telles que la triéthylamine, la tributylamine, la pyridine, la tris(dioxa-3,6 heptyl)amine, le diaza-1,8 bicyclo(5,4,0)undécène-7.

On peut utiliser également des composés aminés comme l'hydrazine ou ses dérivés, notamment les dérivés obtenus par substitution d'un ou de deux atomes d'hydrogène par des radicaux alkyles, aryles, cycloaliphatiques ou hétérocycliques.

La quantité d'amine utilisée dans le procédé peut varier dans de très larges limites.

Habituellement elle représente en poids par rapport au poids du chlorophénol à chlorer de 0,005 % à 25 %.

On mettra en oeuvre préférentiellement de 0,015 % à 5 % en poids d'amine par rapport au chlorophénol, afin d'avoir une efficacité suffisante, tout en n'ayant pas une quantité excessive d'amine.

Parmi les amines de formule générale (V), on préfère dans le cadre du présent procédé, les amines primaires ou secondaires de formule (VI) :

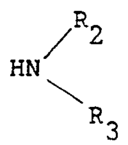

(VI)

dans laquelle :

- $R_2$ ou $R_3$ représente un atome d'hydrogène ;

- $R_2$ et $R_3$, identiques ou différents, représentent :

. un radical alkyle linéaire ayant 1 à 10 atomes de carbone ;

. un radical alkyle secondaire ayant 3 à 10 atomes de carbone ;

. un radical alkyle tertiaire ayant 4 à 10 atomes de carbone ;

. un radical cyclohexyle ou cyclopentyle ;

. un radical phényle ;

. un radical benzyle ou phénéthyle ;

- $R_2$ et $R_3$ forment ensemble, avec l'atome d'azote et avec un autre atome d'azote et/ou d'oxygène, un hétérocycle saturé ou comportant une ou plusieurs insaturations ;

- $R_2$ et/ou $R_3$ comportent une ou plusieurs fonctions amine, hydroxyle ou ester d'acide carboxylique.

A titre d'exemples d'amines primaires de formule générale (VI), on peut citer la n-propylamine, l'isopropylamine, la n-butylamine, l'isobutylamine, la tertiobutylamine, la n-pentylamine, la méthyl-2 pentylamine, la méthyl-3 pentylamine, l'éthyl-2 hexylamine, la laurylamine, la cyclohexylamine, la cyclopentylamine, la benzylamine, l'ester éthylique de la glycine et l'éthanolamine.

En ce qui concerne plus particulièrement les amines secondaires de formule générale (VI), on préfère encore plus spécialement celles pour lesquelles au moins un des symboles $R_2$ et $R_3$ et de préférence les deux symboles $R_2$ et $R_3$ représentent :

. un radical alkyle secondaire ayant de 3 à 10 atomes

de carbone tel qu'isopropyle, butyle-2, pentyle-2, pentyle-3, hexyle-2, hexyle-3, heptyle-2, heptyle-3, heptyle-4, octyle-2, octyle-3, octyel-4, nonyle-2, nonyle-3, nonyle-4, nonyle-5, décyle-2, décyle-3, décyle-4 et décyle-5 ;
. un radical cyclohexyle ou cyclopentyle ;

et celles pour lesquelles $R_2$ et $R_3$ forment avec l'atome d'azote un hétérocycle comportant éventuellement un autre atome d'azote ou un atome d'oxygène.

A titre d'exemples de telles amines secondaires, on peut citer la diisopropylamine, la diisobutylamine, la dicyclohexylamine, la morpholine, l'imidazole.

Les sulfures organiques utilisables dans le présent procédé sont plus particulièrement les composés de formule générale (VII) :

$R_5 - S - R_4$ (VII)

dans laquelle $R_4$ et $R_5$ identiques ou différents, représentent :
- un radical phényle, éventuellement substitué par un ou plusieurs radicaux alkyles, alkoxy, hydroxyle ou atomes de chlore ;
- un radical alkyle ;
- un radical phénylalkyle, éventuellement substitué par un ou plusieurs radicaux alkyles, alkoxy, hydroxyle ou atomes de chlore ;
- un radical cyclopentyle ou cyclohexyle, éventuellement substitué par un ou plusieurs radicaux alkyles, alkoxy, hydroxyle ou atomes de chlore ;
- les symboles $R_4$ et $R_5$ lorsqu'ils représentent des radicaux phényles peuvent être reliés entre eux par un atome de soufre.

A titre d'exemples de tels sulfures organiques, on peut citer le thioanisole, le sulfure de diphényle, le sulfure de phényle et de chloro-4 phényle, le sulfure de di(chloro-4 phényle), le sulfure de di(hydroxy-2 ditertiobutyl-4,6 phényle), le sulfure de di(hydroxy-4 diméthyl-3,5 phényle) le sulfure de di(hydroxy-2 ditertiobutyl-3,5 phényle), le sulfure de phényle et de cyclohexyle, le sulfure de phényle et de butyle, le sulfure de chloro-4 phényle et d'hexyle, le sulfure de méthyl-4 phényle et de dodécyle, le sulfure de benzyle et d'octyle, le sulfure de méthoxy-4 phényle et d'éthyle, le sulfure d'éthyle et d'hexyle, le sulfure de dioctyle, le sulfure de propyle et de méthyl-4 cyclohexyle, le sulfure de méthyl et de chloro-4 cyclohexyle, le thianthrène.

L'utilisation conjointe d'un cation organique ou d'une amine tels que définis prédemment et d'un sulfure organique, permet d'obtenir le trichloro-2,4,6 phénol avec très peu de chlorocyclohexadiènone ou d'autres sous-produits indésirables. En outre la réaction entre le chlore gazeux et le chlorophénol est pratiquement totale.

La quantité de sulfure organique que l'on peut utiliser dans le présent procédé peut varier largement.

Habituellement elle représente en poids par rapport au poids de chlorophénol à chlorer de 0,1 % à 10 %.

De préférence cette quantité représentera en poids par rapport au poids de chlorophénol de 0,5 % à 5 %.

Le sulfure organique peut être introduit dans le milieu réactionnel en même temps que le cation organique ou l'amine, dès le début de la réaction. Il peut aussi être introduit plus tard, au cours de la réaction de chloration.

Le procédé de l'invention s'applique aux monochlorophénols et aux dichlorophénols indiqués précédemment, pris isolément ou en mélanges.

On peut également utiliser comme produits de départ des mélanges industriels contenant, outre des proportions variables de ces chlorophénols, des traces de phénol et de trichloro-2,4,6 phénol.

La quantité de chlore utilisée dans le procédé de l'invention est par conséquent essentiellement fonction du chlorophénol ou du mélange de chlorophénols mis en oeuvre.

Le chlore peut être utilisé seul ou dilué par un gaz inerte, tel que l'azote par exemple. La présence d'un gaz inerte permet, si nécessaire, d'augmenter le débit gazeux sans augmenter la quantité de chlore introduite en un temps donné.

La température à laquelle est mis en oeuvre le procédé est généralement inférieure ou égale à 150 ° C. D'autre part la limite inférieure de la zone de température est conditionnée par le point de fusion du chlorophénol ou du mélange de chlorophénols utilisé.

Généralement cette température est comprise entre le point de fusion du mélange réactionnel et 120° C, mais sans que ces chiffres aient une valeur critique.

Les exemples qui suivent illustrent le procédé de l'invention.

EXEMPLE 1

Dans un ballon en verre de 250 $cm^3$, muni d'une gaine thermométrique, d'une agitation à palettes, d'un réfrigérant et d'un tube plongeant pour l'arrivée du chlore, on charge :
- dichloro-2,4 phénol 40,75 g (250 mmol)
- diisopropylamine 0,04 g (0,1 % en poids)
- thionisole 0,40 g (1 % en poids)

On chauffe à 70° C et on introduite le chlore avec un débit de 5 litres/heure en 1h14 min. On introduite en tout 6,16 litres de chlore (275 mmol).

On effectue alors un prélèvement que l'on dose par chromatographie en phase gazeuse (CPG) et par chromatographie liquide haute performance (CLHP).

On obtient les résultats suivants :

- taux de transformation (TT) du dichloro-2,4 phénol : 100 %
- rendement en trichloro-2,4,6 phénol (RT) : 97,6 %
- RT en tétrachloro-2,3,4,6 phénol : 2,0 %
- RT en tétrachloro-2,4,4,6 cyclohexadiènone : 0,45 %
- dichloro-2,6 benzoquinone : moins de 0,1 %
- tétrachlorobenzoquinone : moins de 0,1 %.

On pousuit le chauffage à 70° C pour le reste de l'essai pendant 2 heures sans injecter de chlore.

En fin d'essai, on refroidit et on purge à l'azote. On dose le milieu réactionnel final comme précédemment.

On obtient les mêmes résultats que pour l'échantillon prélevé mais le RT en tétrachloro-2,4,4,6 cyclohexadiènone passe à 0,2 %.

Un essai témoin effectué dans les mêmes conditions que dans la première partie de l'exemple 1, mais sans thionisole a conduit aux résultats suivants :

- taux de transformation (TT) du dichloro-2,4 phénol : 100 %
- rendement en trichloro-2,4,6 phénol (RT) : 96,8 %
- RT en tétrachloro-2,3,4,6 phénol : 1,2 %
- RT en tétrachloro-2,4,4,6 cyclohexadiènone : 1,3 %

EXEMPLE 2

On répète l'exemple 1 avec les mêmes charges de réactifs, mais après une heure d'injection du chlore on rajoute à nouveau 0,4 g de thioanisole. En fin d'injection de chlore (1h14 min), on chauffe pendant 2 heures à 70° C sans chlore.

On traite et on dose l'essai comme précédemment.

On obtient les résultats suivants :

- taux de transformation (TT) du dichloro-2,4 phénol : 100 %
- rendement en trichloro-2,4,6 phénol (RT) : 97,4 %
- RT en tétrachloro-2,3,4,6 phénol : 2,0 %
- RT en tétrachloro-2,4,4,6 cyclohexadiènone : pas de trace
- pas de trace de chlorobenzoquinones.

EXEMPLE 3

On répète l'exemple 1 avec les différences suivantes :

- le thianthrène à la place du thionanisole
- addition du thianthrène après 1 heure d'injection du chlore
- poursuite du chauffage à 70° C pendant 1h30 min après la fin d'injection du chlore.

Après dosages, on obtient les résultas suivants :

- taux de transformation (TT) du dichloro-2,4 phénol : 100 %
- rendement en trichloro-2,4,6 phénol (RT) : 95,0 %
- RT en tétrachloro-2,3,4,6 phénol : 2,0 %
- RT en tétrachloro-2,4,4,6 cyclohexadiènone : pas de trace
- pas de trace de chlorobenzoquinones.

**Revendications**

1) Procédé de préparation de trichloro-2,4,6 phénol, par chloration par le chlore gazeux, d'orthochlorophénol et/ou de parachlorophénol et/ou de dichloro-2,4 phénol et/ou de dichloro-2,6 phénol, caractérisé en ce que l'on opère en présence :

- d'une quantité efficace d'un cation organique ou d'une amine primaire, secondaire ou tertiaire ;
- et d'une quantité efficace d'un sulfure organique.

2) Procédé selon la revendication 1, caractérisé en ce que l'on opère en présence d'au moins un cation organique choisi parmi les ions onium répondant à l'une des formules générales suivantes :

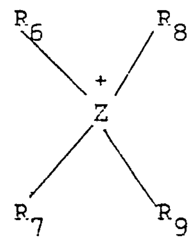

(I)

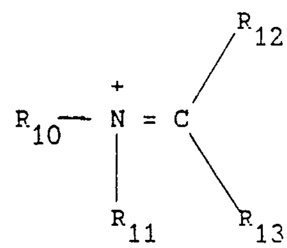

(II)

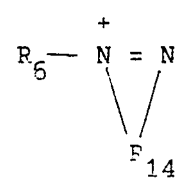

(II bis)

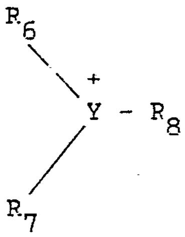

(III)

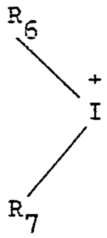

(IV)

dans lesquelles :

- Z représente N, P ou As ;
- Y représente S, O, Se, S = O ou C ;
- $R_6$, $R_7$, $R_8$ et $R_9$, identiques ou différents représentent :

. un radical alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;

. un radical alcényle, linéaire ou ramifié, ayant 2 à 12 atomes de carbone ;

. un radical aryle ayant 6 à 10 atomes de carbone, éventuellement substitué par un ou plusieurs groupements ou atomes alkyles ayant 1 à 4 atomes de carbone, alkoxy, alkoxycarbonyle, le radical alkoxy ayant 1 à 4 atomes de carbone, ou halogène,

. deux desdits radicaux $R_6$ à $R_9$ pouvant former ensemble un radical alkylène, alcénylène ou alcadiènylène, linéaire ou ramifié ayant de 3 à 6 atomes de carbone ;

- $R_{10}$, $R_{11}$, $R_{12}$, $R_{13}$ sont identiques ou différents et représentent :

. un radical alkyle linéaire ou ramifié contenant de 1 à 4 atomes de carbone ;

. les radicaux $R_{12}$ et $R_{13}$ pouvant former ensemble un radical alkylène contenant de 3 à 6 atomes de carbone ;

. les radicaux $R_{11}$ et $R_{12}$ ou $R_{11}$ et $R_{13}$ pouvant former ensemble un radical alkylène, alcénylène ou alcadiènylène, contenant 4 atomes de carbone et constituant avec l'atome d'azote un hétérocycle azoté ;

- $R_{14}$ représente un radical divalent formant avec les 2 atomes d'azote un cycle ayant 4 à 6 atomes pouvant comporter un ou plusieurs atomes d'azote, de soufre et/ou d'oxygène ledit cycle pouvant être substitué par un ou plusieurs radicaux tels que $R_6$.

3) Procédé selon l'une des revendications 1 ou 2, caractérisé en ce que l'anion lié aux cations organiques est choisi parmi les ions : $F^-$, $ClO_4^-$, $PF_6^-$, $BF_4$, $SnCl_6^-$, $SbCl_6^-$, $B(Ph)_4^-$, $PO_4^{3-}$, $HPO_4^{2-}$, $H_2PO_4^-$, $CH_3SO_3^-$, $Ph\text{-}SO_3^-$, $HSO_4^-$, $NO_3^-$, $SO_4^{2-}$, $Cl^-$, $Br^-$, $I^-$, $OH^-$, Ph représentant un radical phényle.

4) Procédé selon l'une des revendications 1 ou 2, caractérisé en ce que l'anion lié aux cations organiques est choisi parmi les ions : $PO_4^{3-}$, $HPO_4^{2-}$, $H_2PO_4^-$, $CH_3SO_3^-$, $Ph\text{-}SO_3^-$, $NO_3^-$, $SO_4^{2-}$, $PF_6^-$, $Cl^-$, $Br^-$, $I^-$, Ph représentant un radical phényle.

5) Procédé selon l'une des revendications 1 à 4, caractérisé en ce que le cation organique est présent en quantité telle que le poids de sel d'onium par rapport au poids de chlorophénol à chlorer varie de 0,005 % à 25 %.

6) Procédé selon l'une des revendications 1 à 5, caractérisé en ce que l'on opère à l'état fondu en présence de 0,015 % à 5 % en poids de sel d'onium par rapport au chlorophénol à chlorer.

7) Procédé selon l'une des revendications 1 à 5, caractérisé en ce que l'on opère en milieu solvant et en présence de 5 % à 25 % en poids de sel d'onium par rapport au chlorophénol à chlorer.

8) Procédé selon la revendication 7, caractérisé en ce que le solvant constitué par un éther aliphatique, un hydrocarbure aliphatique, un hydrocarbure aliphatique chloré, un chlorbenzène ou un bromobenzène, un acide carboxylique.

9) Procédé selon la revendication 1, caractérisé en ce que l'amine utilisée répond à la formule générale (V) :

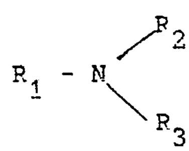

(V)

dans laquelle :

- $R_1$, $R_2$ et $R_3$, identiques ou différents, représentent :

. un radical alkyle linéaire ayant de 1 à 12 atomes de carbone, un radical alkyle secondaire ayant 3 à 12 atomes de carbone ou un radical alkyle tertiaire ayant 4 à 12 atomes de carbone, ces radicaux alukyles pouvant comporter une ou deux fonctions éther -O- ou fonctions hydroxyle, amine, acide carboxylique, ester d'acide carboxylique, amide ou imine ;

. un radical phényle, un radical cyclohexyle, un radical cycloheptyle ou un radical cyclopentyle ;

. un radical phénylalkyle, cyclohexylalkyle, cycloheptylalkyle ou cyclo pentylalkyle dont la partie alkyle comporte 1 à 4 atomes de carbone ;

. un atome d'hydrogène.

- $R_1$ peut représenter un groupement $NH_2$ ;
- $R_2$ et $R_3$ forment ensemble et avec l'atome d'azote un hétérocycle, saturé ou comportant

une ou plusieurs doubles liaisons, éventuellement substitué par un ou plusieurs groupements alkyles ayant 1 à 4 atomes de carbone ;

- $R_2$ et $R_3$ ou $R_1$, $R_2$ et $R_3$ forment ensemble, avec l'atome d'azote et avec un ou plusieurs atomes d'azote et/ou d'oxygène et/ou de soufre, un hétérocycle, saturé ou insaturé, éventuellement substitué par un ou plusieurs groupements alkyles ayant 1 à 4 atomes de carbone ;
- $R_1$, $R_2$ et $R_3$ forment ensemble et avec l'atome d'azote un hétérocycle insaturé éventuellement substitué par un ou deux groupements méthyle ou éthyle ;
- $R_2$ et $R_3$ ou $R_1$, $R_2$ et $R_3$ forment ensemble, avec l'atome d'azote et éventuellement avec un ou plusieurs atomes d'azote et/ou d'oxygène et/ou de soufre, un composé polycyclique, saturé ou insaturé, éventuellement substitué par un ou plusieurs groupements alkyles ayant 1 à 4 atomes de carbone.

10) Procédé selon l'une des revendications 1 ou 9, caractérisé en ce que l'amine utilisée répond à la formule générale (VI)

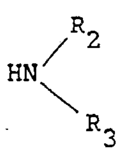

(VI)

dans laquelle :

- $R_2$ ou $R_3$ représente un atome d'hydrogène ;
- $R_2$ et $R_3$, identiques ou différents, représentent :
  . un radical alkyle linéaire ayant 1 à 10 atomes de carbone ;
  . un radical alkyle secondaire ayant 3 à 10 atomes de carbone ;
  . un radical alkyle tertiaire ayant 4 à 10 atomes de carbone ;
  . un radical cyclohexyle ou cyclopentyle ;
  . un radical phényle ;
  . un radical benzyle ou phénéthyle ;
- $R_2$ et $R_3$ forment ensemble, avec l'atome d'azote et avec un autre atome d'azote et/ou d'oxygène, un hétérocycle saturé ou comportant une ou plusieurs insaturations ;
- $R_2$ et/ou $R_3$ comportent une ou plusieurs fonctions amine, hydroxyle ou ester d'acide carboxylique.

11) Procédé selon l'une des revendications 1, 9 ou 10, caractérisé en ce que l'amine est choisi parmi : la n-propylamine, l'isopropylamine, la n-butylamine, l'isobutylamine, la tertiobutylamine, la n-pentylamine, la méthyl-2 pentylamine, la méthyl-3 pentylamine, l'éthyl-2 hexylamine, la laurylamine, la cyclohexylamine, la cyclopentylamine, la benzylamine, l'ester éthylique de la glycine, l'éthanolamine, la diisopropylamine, la diisobutylamine, la dicyclohexylamine, la morpholine, 1'imidazole.

12) Procédé selon l'une des revendications 1, 9, 10 ou 11, caractérisé en ce que l'amine utilisée représente en poids par rapport au poids du chlorophénol à chlorer de 0,005 % à 25 % et de préférence de 0,015 % à 5 %.

13) Procédé selon la revendication 1, caractérisé en ce que l'on utilise un sulfure organique de formule générale (VII) :

$R_5 - S - R_4$ (VII)

dans laquelle $R_4$ et $R_5$ identiques ou différents, représentent :

- un radical phényle, éventuellement substitué par un ou plusieurs radicaux alkyles, alkoxy, hydroxyle ou atomes de chlore ;
- un radical alkyle ;
- un radical phénylalkyle, éventuellement substitué par un ou plusieurs radicaux alkyles, alkoxy, hydroxyle ou atomes de chlore ;
- un radical cyclopentyle ou cyclohexyle, éventuellement substitué par un ou plusieurs radicaux alkyles, alkoxy, hydroxyle ou atomes de chlore ;
- les symboles $R_4$ et $R_5$ lorsqu'ils représentent des radicaux phényles peuvent être reliés entre eux par un atome de soufre.

14) Procédé selon l'une des revendications 1 ou 13, caractérisé en ce que le sulfure organique est choisi parmi : le thioanisole, le sulfure de diphényle, le sulfure de phényle et de chloro-4 phényle, le sulfure de di(chloro-4 phényle), le sulfure de di(hydroxy-2 ditertiobutyl-4,6 phényle), le sulfure de di(hydroxy-4 diméthyl-3,5 phényle) le sulfure de di(hydroxy-2 ditertiobutyl-3,5 phényle), le sulfure de phényle et de cyclohexyle, le sulfure de phényle et de butyle, le sulfure de chloro-4 phényle et d'hexyle, le sulfure de méthyl-4 phényle et de dodécyle, le sulfure de benzyle et d'octyle, le sulfure de méthoxy-4 phényle et d'éthyle, le sulfure d'éthyle et d'hexyle, le sulfure de dioctyle, le sulfure de propyle et de méthyl-4 cyclohexyle, le sulfure de méthyl et de chloro-4 cyclohexyle, le thianthrène.

15) Procédé selon l'une des revendications 1, 13 ou 14, caractérisé en ce que le sulfure organique représente en poids par rapport au chlorophénol à chlorer de 0,1 % à 10 % et de préférence de 0,5 % à 5 %.

Office européen des brevets

# RAPPORT DE RECHERCHE EUROPEENNE

Numero de la demande

EP 88 42 0244

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | PATENT ABSTRACTS OF JAPAN, vol. 11, no. 43 (C-402)[2490], 7 février 1987, page 29 C 402; & JP-A-61 207 351 (INUIU YAKUHIN KOGYO K.K.) 13-09-1986 | 1,2 | C 07 C 37/62 |
| A | FR-A-2 584 068 (RHONE-POULENC) * Revendications 1-10 * | 1,2 | |
| A | FR-A-2 307 785 (DOW CHEMICAL CO.) * Revendications 1,8 * | 1 | |
| A | EP-A-0 196 260 (RHONE-POULENC) * Revendications 1-15 * | 1,11,12 | |
| A | US-A-4 223 166 (JAEGER et al.) * Revendication 1 * | 1,2 | |
| | | | DOMAINES TECHNIQUES RECHERCHES (Int. Cl.4) |
| | | | C 07 C 37/00<br>C 07 C 39/00 |

Le présent rapport a été établi pour toutes les revendications

| Lieu de la recherche | Date d'achèvement de la recherche | Examinateur |
|---|---|---|
| LA HAYE | 11-10-1988 | KLAG M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)